(19) Europäisches Patentamt
European Patent Office
Office européen des brevets

(11) Veröffentlichungsnummer: **0 170 860**
**A1**

(12) **EUROPÄISCHE PATENTANMELDUNG**

(21) Anmeldenummer: **85107933.5**

(22) Anmeldetag: **26.06.85**

(51) Int. Cl.⁴: **C 07 D 471/14**, C 07 F 7/10, A 01 N 43/90 // (C07D471/14, 235:00, 221:00, 209:00)

(30) Priorität: **07.07.84 DE 3425124**
**07.06.85 DE 3520390**

(43) Veröffentlichungstag der Anmeldung: **12.02.86**
**Patentblatt 86/7**

(84) Benannte Vertragsstaaten: **BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Draber, Wilfried, Dr., In den Birken 81, D-5600 Wuppertal 1 (DE)**
Erfinder: **Eue, Ludwig, Dr., Paul-Klee-Strasse 36, D-5090 Leverkusen 1 (DE)**
Erfinder: **Santel, Hans-Joachim, Dr., Gerstenkamp 19, D-5000 Köln 80 (DE)**
Erfinder: **Schmidt, Robert R. Dr., Im Waldwinkel 110, D-5060 Bergisch-Gladbach 2 (DE)**

(54) **Imidazo-pyrrolo-pyridin-Derivate.**

(57) Die Erfindung betrifft neue Imidazo-pyrrolo-pyridin-Derivate der allgemeinen Formel (I),

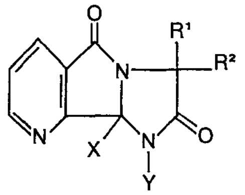

(I)

in welcher
$R^1$ und $R^2$ unabhängig voneinander für Alkyl stehen,
X für Cyano, Alkoxycarbonyl oder Aminocarbonyl steht und
Y für Wasserstoff steht oder für den Fall, daß X für Cyano steht auch für die Trimethylsilylgruppe steht,
Verfahren zu ihrer Herstellung und ihre Verwendung als Herbizide.

EP 0 170 860 A1

BAYER AKTIENGESELLSCHAFT 5090 Leverkusen, Bayerwerk
Konzernverwaltung RP
Patentabteilung KM/Kü-c
Ia

## Imidazo-pyrrolo-pyridin-Derivate

Die Erfindung betrifft neue Imidazo-pyrrolo-pyridin-Derivate, Verfahren zu ihrer Herstellung und ihre Verwendung als Herbizide.

Es ist bereits bekannt, daß bestimmte 2-(2-Imidazolin-2-yl)-pyridine, wie beispielsweise das 2-(4,4-Dimethyl-5-oxo-2-imidazolin-2-yl)-3-methoxycarbonyl-pyridin, herbizide Eigenschaften haben (vergl. z. B. EP-OS 41 623).

Die herbizide Wirkung dieser vorbekannten Verbindungen gegenüber Schadpflanzen, ebenso wie ihre Verträglichkeit gegenüber wichtigen Kulturpflanzen ist jedoch nicht immer in allen Anwendungsbereichen völlig zufriedenstellend.

Es wurden neue Imidazo-pyrrolo-pyridin-Derivate der allgemeinen Formel (I),

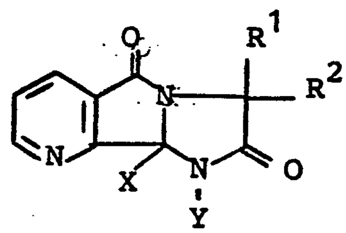

(I)

in welcher

$R^1$ und $R^2$ unabhängig voneinander für Alkyl stehen,

X für Cyano, Alkoxycarbonyl oder Aminocarbonyl steht und

Y für Wasserstoff steht oder für den Fall, daß X für Cyano steht auch für die Trimethylsilylgruppe steht,

gefunden.

Weiterhin wurde gefunden, daß man die neuen Imidazo-pyrrolo-pyridin-Derivate der Formel (I),

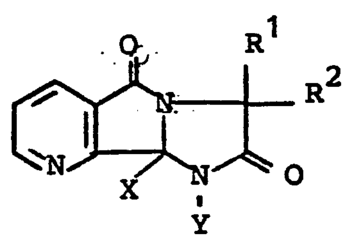

(I)

in welcher

$R^1$ und $R^2$ unabhängig voneinander für Alkyl stehen,
X für Cyano, Alkoxycarbonyl oder Aminocarbonyl steht und
Y für Wasserstoff steht oder für den Fall, daß X für Cyano steht auch für die Trimethylsilylgruppe steht,

erhält, wenn man

(a) Imidazo - pyrrolo-pyridine der Formel (II)

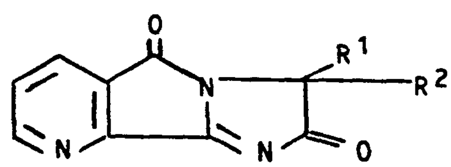

(II)

in welcher

$R^1$ und $R^2$ die oben angegebene Bedeutung haben,

mit Trimethylsilylcyanid der Formel (III),

$$(CH_3)_3Si - CN \qquad (III)$$

gegebenenfalls in Gegenwart eines Verdünnungsmittels umsetzt oder wenn man

(b) die nach Verfahren (a) erhältlichen Imidazo-pyrrolo-trimethylsilyl-pyridine der Formel (Ia),

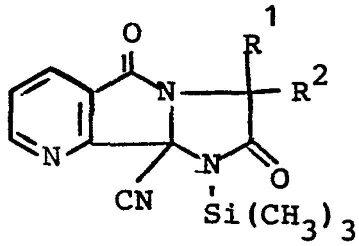

(Ia)

in welcher

$R^1$ und $R^2$ die oben angegebene Bedeutung haben,

durch Umsetzung mit Hydroxyverbindungen der Formel (IV),

$$R^3 - OH \qquad (IV)$$

in welcher

$R^3$ für Wasserstoff oder für Alkyl steht,

gegebenenfalls in Gegenwart eines Verdünnungsmittels und gegebenenfalls in Gegenwart eines Katalysators verseift.

Schließlich wurde gefunden, daß die neuen Imidazo-pyrrolo-pyridin-Derivate der Formel (I) herbizide, insbesondere auch selektiv-herbizide Eigenschaften besitzen.

Überraschenderweise zeigen die erfindungsgemäßen Imidazo-pyrrolo-pyridin-Derivate der Formel (I) bei vergleichbar guter Nutzpflanzenverträglichkeit eine erheblich höhere herbizide Potenz als die aus dem Stand der Technik bekannten 2-(2-Imidazolin-2-yl)-pyridine, wie beispielsweise das 2-(4,4-Dimethyl-5-oxo-2-imidazolin-2-yl)-3-methoxycarbonyl-pyridin, welche chemisch und wirkungsmäßig naheliegende Verbindungen sind.

Die erfindungsgemäßen Imidazo-pyrrolo-pyridin-Derivate sind durch die Formel (I) allgemein definiert. Bevorzugt sind Verbindungen der Formel (I), bei welchen

$R^1$ und $R^2$ unabhängig voneinander für geradkettiges oder verzweigtes Alkyl mit 1 bis 8 Kohlenstoffatomen stehen,

X für Cyano, Aminocarbonyl oder geradkettiges oder verzweigtes Alkoxycarbonyl mit 1 bis 8 Kohlenstoffatomen steht und

Y für Wasserstoff steht oder für den Fall, daß X für Cyano steht, auch für die Trimethylsilylgruppe steht.

Besonders bevorzugt sind Verbindungen der Formel (I), bei welchen

$R^1$ und $R^2$ unabhängig voneinander für Methyl, Ethyl, n- oder i-Propyl sowie n-, i-, s- oder t-Butyl stehen,

X für Cyano, Aminocarbonyl, Methoxycarbonyl, Ethoxycarbonyl, n- oder i-Propoxycarbonyl, sowie für n-, i-, s- oder t-Butoxycarbonyl steht und

Y für Wasserstoff steht oder für den Fall, daß X für Cyano steht, auch für die Trimethylsilylgruppe steht.

Im einzelnen seien die bei den Herstellungsbeispielen aufgeführten Verbindungen der allgemeinen Formel (I) genannt.

Verwendet man beispielsweise die folgenden Verbindungen als Ausgangsstoffe, so läßt sich der Reaktionsablauf des erfindungsgemäßen Verfahrens (a) durch das folgende Formelschema darstellen:

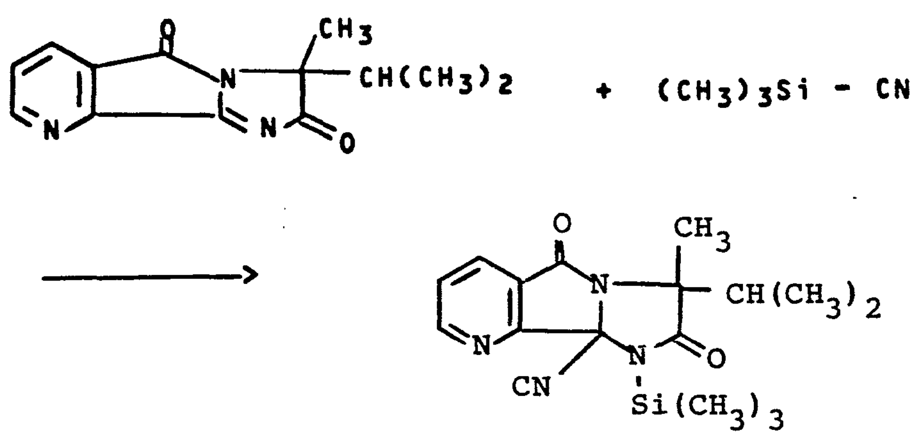

Verwendet man beispielsweise die folgenden Verbindungen als Ausgangsstoffe, so läßt sich der Reaktionsablauf des erfindungsgemäßen Verfahrens (b) durch das folgende Formelschema darstellen:

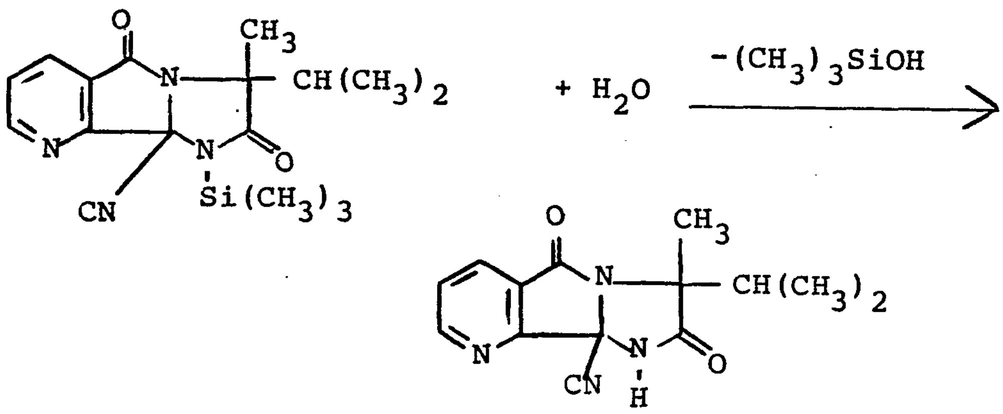


Verwendet man beispielsweise die folgenden Verbindungen als Ausgangsstoffe und Schwefelsäure als Katalysator, so läßt sich der Reaktionsablauf des erfindungsgemäßen Verfahrens (b) durch das folgende Formelschema darstellen:

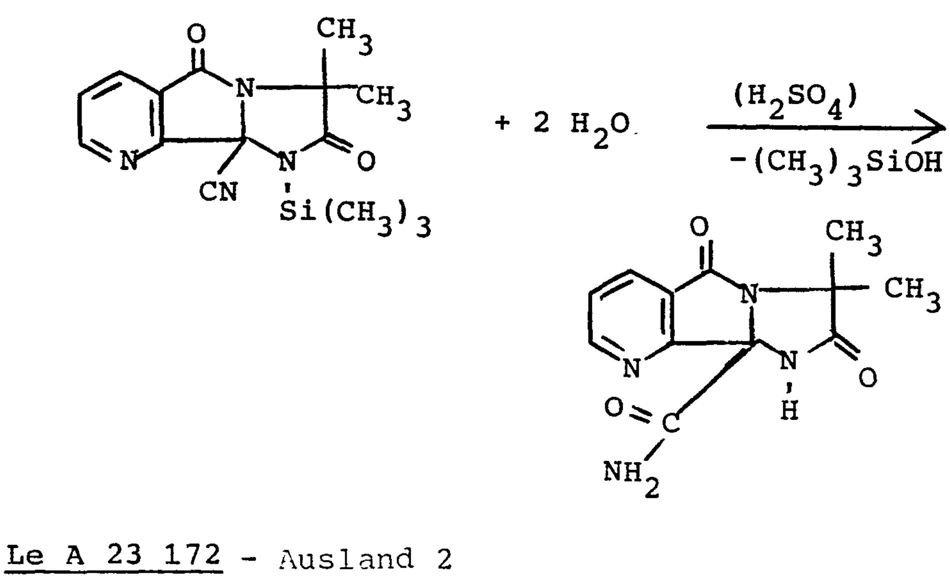

Verwendet man beispielsweise die folgenden Verbindungen als Ausgangsstoffe und Chlorwasserstoff als Katalysator, so läßt sich der Reaktionsablauf des erfindungsgemäßen Verfahrens (b) durch das folgende Formelschema darstellen:

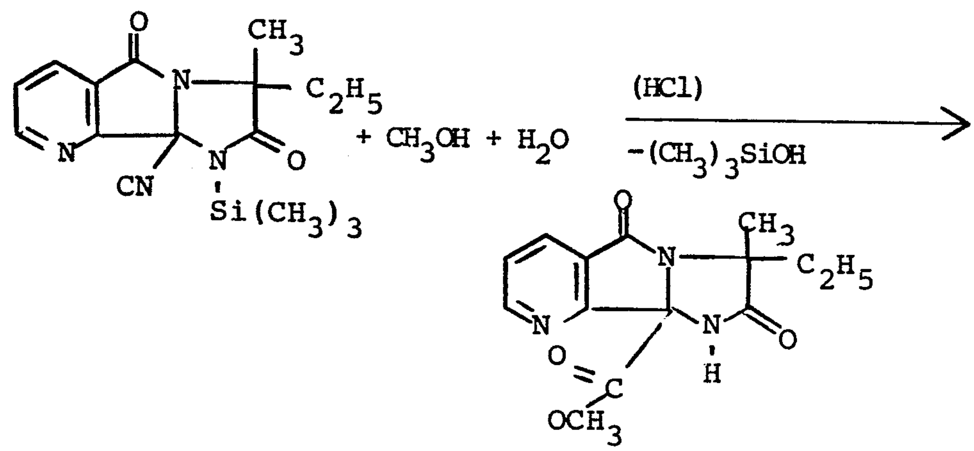


Die zur Durchführung des erfindungsgemäßen Verfahrens (a) als Ausgangsstoffe benötigten Imidazo-pyrrolopyridine sind durch die Formel (II) allgemein definiert. In dieser Formel (II) stehen $R^1$ und $R^2$ vorzugsweise für diejenigen Reste, die schon im Zusammenhang mit der Beschreibung der erfindungsgemäßen Stoffe der Formel (I) als bevorzugt für diese Substituenten genannt wurden.

Die Imidazo-pyrrolo-pyridine der Formel (II) und Verfahren zu ihrer Herstellung sind bekannt (vergl. z. B. EP-OS 41 623).

Das zur Durchführung des erfindungsgemäßen Verfahrens (a) weiterhin als Ausgangsverbindung benötigte Trimethylsilylcyanid der Formel (III) ist eine allgemein bekannte Verbindung der organischen Chemie (vgl. US-PS 4 328 351).

Die zur Durchführung des erfindungsgemäßen Verfahrens (b) als Ausgangsstoffe benötigten Imidazo-pyrrolo-trimethylsilyl-pyridine sind durch die Formel (Ia) allgemein definiert. In dieser Formel (Ia) stehen $R^1$ und $R^2$ vorzugsweise für diejenigen Reste, die schon im Zusammenhang mit der Beschreibung der erfindungsgemäßen Stoffe der Formel (I) als bevorzugt für diese Substituenten genannt wurden.

Die Imidazo-pyrrolo-trimethylsilyl-pyridine der Formel (Ia) sind erfindungsgemäße Verbindungen und erhältlich nach Verfahren (a).

Die zur Durchführung des erfindungsgemäßen Verfahrens (b) weiterhin als Ausgangsstoffe benötigten Hydroxyverbindungen sind durch die Formel (IV) allgemein definiert. In dieser Formel (IV) steht $R^3$ vorzugsweise für Wasserstoff oder für geradkettiges oder verzweigtes Alkyl mit 1 bis 8 Kohlenstoffatomen, insbesondere für Wasserstoff, Methyl, Ethyl, n- oder i-Propyl oder n-, i-, s- oder t-Butyl.

Die Hydroxyverbindungen der Formel (IV) sind ebenfalls allgemein bekannte Verbindungen der organischen Chemie.

Das erfindungsgemäße Verfahren (a) kann gegebenenfalls in Gegenwart eines geeigneten Verdünnungsmittels durchgeführt werden.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, oder Ether, wie Diethylether, Dioxan, Tetrahydrofuran oder Ethylenglykoldimethyl- oder -diethylether. Auch ein Überschuß des als Reaktionspartner verwendeten Trimethylsilylcyanids der Formel (III), welcher gleichzeitig als Verdünnungsmittel dient, ist möglich.

Die Reaktionstemperaturen können bei der Durchführung des erfindungsgemäßen Verfahrens (a) in einem größeren Bereich variiert werden. Im allgemeinen arbeitet man bei Temperaturen zwischen 20°C und 200°C, vorzugsweise bei Temperaturen zwischen 50°C und 150°C.

Zur Durchführung des erfindungsgemäßen Verfahrens (a) setzt man pro Mol Imidazo-pyrrolo-pyridin der Formel (II) im allgemeinen 1,0 bis 30,0 Mol, vorzugsweise 1,0 bis 15,0 Mol, an Trimethylsilylcyanid ein.

Die Reaktionsmischung wird für mehrere Stunden bis Tage bei der erforderlichen Temperatur gerührt und zur Isolierung der Reaktionsprodukte der Formel (Ia) im Vakuum von allen flüchtigen Bestandteilen befreit. Die Charakterisierung und Identifizierung erfolgt durch spektroskopische Methoden, beispielsweise durch IR- oder NMR-Spektroskopie oder Röntgenstrukturanalyse.

Das erfindungsgemäße Verfahren (b) kann ebenfalls gegebenenfalls in Gegenwart eines geeigneten Verdünnungsmittels durchgeführt werden.

Als solche kommen vorzugsweise aliphatische oder aromatische, gegebenenfalls halogenierte Kohlenwasserstoffe, wie beispielsweise Benzin, Benzol, Toluol, Xylol, Chlorbenzol, Petrolether, Ligroin, Hexan, Cyclohexan, Dichlormethan, Chloroform oder Tetrachlorkohlenstoff infrage.

Es ist jedoch auch möglich, die als Reaktionskomponenten verwendeten Hydroxyverbindungen der Formel (IV) in entsprechendem Überschuß als Verdünnungsmittel einzusetzen.

Zur Durchführung des erfindungsgemäßen Verfahrens (b) kann gegebenenfalls der Zusatz eines Katalysators erforderlich sein. Als solche verwendet man vorzugsweise starke anorganische Säuren, wie Schwefelsäure oder Chlorwasserstoffsäure, gegebenenfalls in Gegenwart eines entsprechenden Salzes wie beispielsweise Natriumchlorid.

Die Reaktionstemperaturen können bei der Durchführung des erfindungsgemäßen Verfahrens (b) ebenfalls in einem größeren Bereich variiert werden. Im allgemeinen arbeitet man bei Temperaturen zwischen 0°C und 150°C, vorzugsweise bei Temperaturen zwischen 20°C und 100°C.

Zur Durchführung des erfindungsgemäßen Verfahrens (b) setzt man pro Mol Imidazo-pyrrolo-trimethylsilyl-

0170860

pyridin der Formel (Ia) im allgemeinen 1,0 bis 30,0 Mol, vozugsweise 1,0 bis 15,0 Mol an Hydroxyverbindungen der Formel (IV) und gegebenenfalls 0,1 bis 10,0 Mol an Katalysatorsäure ein.

In Abhängigkeit von der Säurestärke und -konzentration sowie in Abhängigkeit von der Reaktionstemperatur und der Reaktionsdauer erreicht man bei der Durchführung des erfindungsgemäßen Verfahrens (b) entweder die selektive Abspaltung der Trimethylsilylgruppe oder die gleichzeitige Verseifung der Nitrilfunktion der Verbindungen der Formel (Ia). Bei der Verseifung der Nitrilfunktionen ist es wiederum möglich, durch geeignete Wahl der Reaktionsbedingungen (Katalysator, Temperatur, Reaktionsdauer), eine partielle Verseifung bis zur Amidstufe oder eine vollständige Verseifung zur Carbonsäurestufe mit gleichzeitiger Veresterung der freien Carbonsäuregruppe zu erreichen. Es ist auch möglich die Abspaltung der Trimethylsilylgruppe und die anschließende Nitrilverseifung in zwei getrennten Reaktionsschritten durchzuführen, mit intermediärer Isolierung der entsprechenden Verbindung der Formel (Ib).

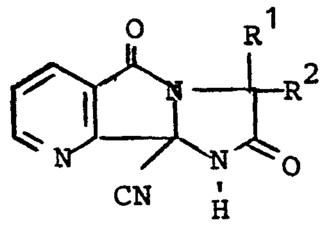

(Ib)

Die Aufarbeitung und Isolierung der jeweiligen Endprodukte der Formel (I) erfolgt nach allgemein üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria,

Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Vor- und Nachauflaufunkrautbekämpfung von mono- und dikotylen Unkräutern in wichtigen vorzugsweise dikotylen Kulturen, wie beispielsweise Baumwolle oder Soja, einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit weiteren Triazinonen oder Harnstoffen oder auch Diphenylether, wie beispielsweise 5-(2-Chlor-4-trifluormethylphenoxy)-2-nitro-benzoesäure oder -N-methyl-sulfonylbenzamid, 5-(2,4-Dichlorphenoxy)-2-nitrobenzoesäure-methylester, 2-[4-(3,5-Dichlorpyrid-2-yloxy)-phenoxy]-propionsäure-(2-benzyloxyethylester) oder -(trimethylsilylmethylester) oder Cyclohexandionen, wie beispielsweise 2-[(1-Ethoxamino)-butyliden]-5-(2-ethylthio-propyl)-1,3-cyclohexandion oder Methyl-6,6-dimethyl-2,4-dioxo-3-[1-(2-propenyloxyamino)-butyliden]-cyclohexancarbonsäure oder Benzothiadiazinonen, wie beispielsweise 3-Isopropyl-2,1,3-benzothiadiazin-4-on-2,2-dioxid sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,05 und 10 kg Wirkstoff pro Hektar Bodenfläche, vorzugsweise zwischen 0,1 und 5 kg pro ha.

Die Herstellung und die Verwendung der erfindungsgemäßen Wirkstoffe geht aus den nachfolgenden Beispielen hervor.

## Herstellungsbeispiele:

### Beispiel 1:

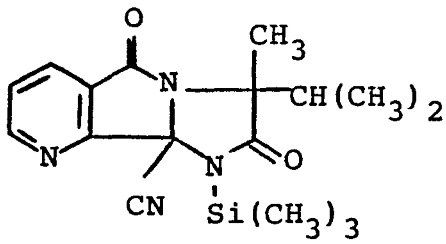

(1)

Eine Mischung von 24,3 g (0,1 Mol) 3-Isopropyl-3-methyl-5H-imidazo-[1',2':1,2]pyrrolo[3,4-b]-pyridin-2-(3H),5-dion und 100 ml (79 g, ca. 1 Mol) Trimethylsilylcyanid wird 16 Stunden lang bei 100°C Badtemperatur gerührt, abgekühlt, eingeengt und die flüchtigen Bestandteile werden im Hochvakuum entfernt. Man erhält 34 g (100 % der Theorie) an Verbindung des Beispiels 1 als Oel.

<u>NMR</u> (60 MHz; ppm/TMS als innerer Standard): 9H, 0.32 ($Si(CH_3)_3$); 11 H, 0.55, 0.12, 1.5, 2.0 ($CH_3$ und $CH(CH_3)_2$): 3H, 7.5, 8.1, 8.9 (Pyridin-Protonen).

<u>MS</u> (chem. Ionisation mit $NH_3$): 343 (M+I), 299 (M+2--$CH(CH_3)_2$), 299 (M+I -$CH(CH_3)_2$-CN).

---

NMR = magnetisches Kernresonanzspektrum (TMS=Tetramethylsilan, $(CH_3)_4Si$));

MS = Massenspektrum (M=Molekülion).

Beispiel 2:

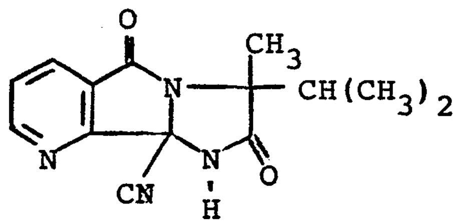

(2)

34,2 g (0,1 Mol) der Verbindung des Beispiels 1, 400 ml Ligroin, 100 ml Methylenchlorid und 2,5 ml (0,14 Mol) Wasser werden für 12 bis 15 Stunden bei Raumtemperatur (20°C bis 25°C) gerührt, filtriert, das Filtrat eingedampft und der Rückstand mit Ether verrührt und abgesaugt. Man erhält 22,5 g (83 % der Theorie) an Verbindung des Beispiels 2 vom Schmelzpunkt 155°C (Zers.).

Beispiel 3:

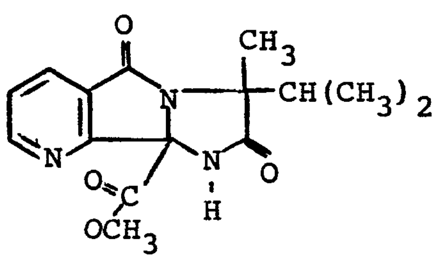

(3)

20,2 g (0,075 Mol) der Verbindung des Beispiels 2

200 ml Methylenchlorid, 12 g (0,375 Mol) Methanol und 40 ml konzentrierte Salzsäure werden 24 Stunden unter Rückfluß gekocht, abgekühlt, im Vakuum eingeengt, der Rückstand in Essigester aufgenommen, mit Wasser und wäßriger Natriumhydrogencarbonatlösung gewaschen, über Natriumsulfat getrocknet und im Vakuum eingeengt. Das zurückbleibende Oel wird über eine Kieselgelsäule (Laufmittel: Chloroform/Essigester/Methanol - 10:10:2) chromatographiert. Man erhält 6,4 g (28 % der Theorie) an Verbindung des Beispiels 3 vom Schmelzpunkt 196°C.

Beispiel 4:

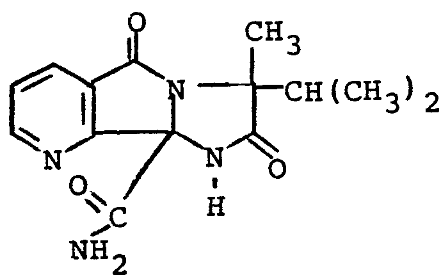

(4)

8,1 g (0,03 Mol) der Verbindung des Beispiels 2,6 g (0,06 Mol) konzentrierte Schwefelsäure, 1 ml (0,055 Mol) Wasser und 0,17 g (0,003 Mol) Natriumchlorid werden für 4 Stunden bei 40°C bis 50°C gerührt. Danach gibt man 50 ml Methanol zu und rührt weitere 3 Stunden bei 50°C und 12 Stunden bei Raumtemperatur (20°C bis 25°C). Zur Aufarbeitung verdünnt man mit 50 ml Wasser, neutralisiert mit Natriumhydrogencarbonat, saugt den ausgefallenen Feststoff ab und trocknet ihn. Man erhält 3,2 g (37 %

der Theorie) an Verbindung des Beispiels 4 vom Schmelzpunkt 281° (Zers.).

In entsprechender Weise und gemäß den allgemeinen Herstellungsangaben erhält man die folgenden Verbindungen der Formel (I):

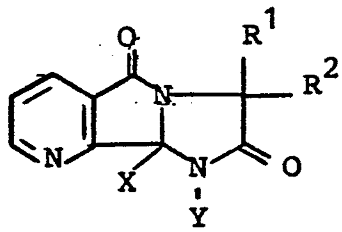

(I)

Beispiel 5: Schmelzpunkt:

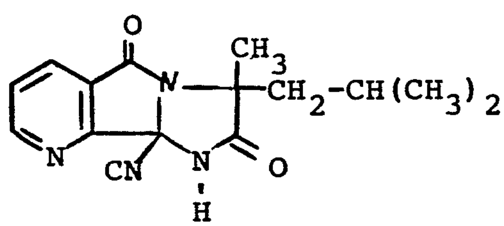

Fp.: 176°C

Beispiel 6:

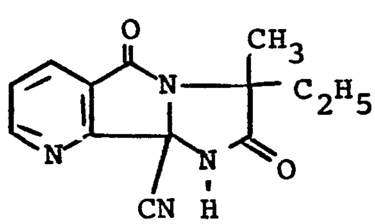

Fp.: 167°C

0170860

Schmelzpunkt:

Beispiel 7:

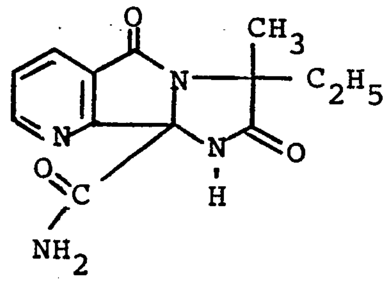

Fp.: 295°C

Beispiel 8:

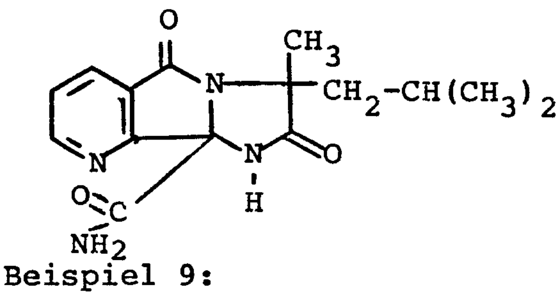

Fp.: 265°C

Beispiel 9:

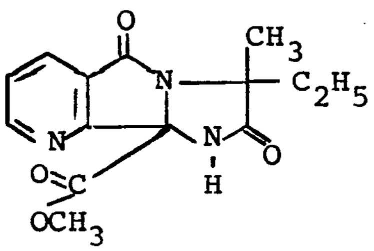

Fp.: 160°C

Beispiel 10:

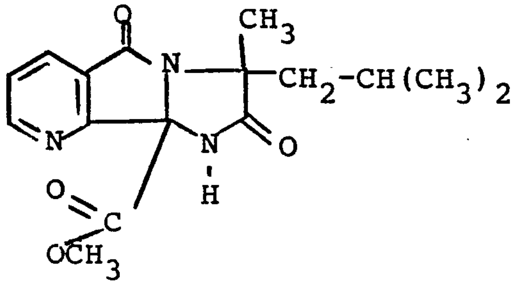

Fp.: 226°C

Schmelzpunkt:

Beispiel 11:

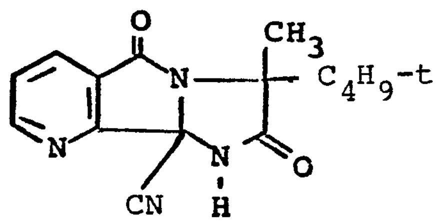

Fp.: 168°C

0170860

Anwendungsbeispiele:

In den folgenden Anwendungsbeispielen wurde die nachstehend aufgeführte Verbindung als Vergleichssubstanz herangezogen:

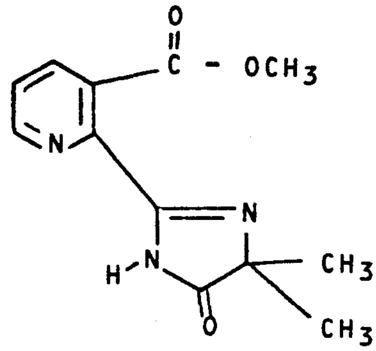

2-(4,4-Dimethyl-5-oxo-2-imidazolin-2-yl)-3-methoxycarbonyl-pyridin.

(bekannt aus EP-OS 41 623)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigt in diesem Test beispielsweise die Verbindung gemäß Herstellungsbeispiel 3.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test beispielsweise die Verbindungen gemäß den Herstellungsbeispielen 1 und 2.

Patentansprüche

1) Imidazo-pyrrolo-pyridin-Derivate der allgemeinen Formel (I),

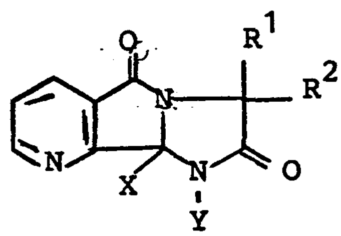

(I)

in welcher

$R^1$ und $R^2$ unabhängig voneinander für Alkyl stehen,
X für Cyano, Alkoxycarbonyl oder Aminocarbonyl steht und
Y für Wasserstoff steht oder für den Fall, daß X für Cyano steht, auch für die Trimethylsilylgruppe steht.

2) Verbindungen der allgemeinen Formel (I) gemäß Anspruch 1, dadurch gekennzeichnet, daß darin

$R^1$ und $R^2$ unabhängig voneinander für geradkettiges oder verzweigtes Alkyl mit 1 bis 8 Kohlenstoffatomen stehen,
X für Cyano, Aminocarbonyl oder geradkettiges oder verzweigtes Alkoxycarbonyl mit 1 bis 8 Kohlenstoffatomen steht und

Y für Wasserstoff steht oder für den Fall, daß X für Cyano steht, auch für die Trimethylsilylgruppe steht.

3) Verbindungen der allgemeinen Formel (I) gemäß Anspruch 1, dadurch gekennzeichnet, daß darin

$R^1$ und $R^2$ unabhängig voneinander für Methyl, Ethyl, n- oder i-Propyl oder n-, i-, s- oder t-Butyl stehen,

X für Cyano, Aminocarbonyl, Methoxycarbonyl, Ethoxycarbonyl, n- oder i-Propoxycarbonyl sowie für n-, i-, s- oder t-Butoxycarbonyl steht und

Y für Wasserstoff steht oder für den Fall, daß X für Cyano steht, auch für die Trimethylsilylgruppe steht.

4) Verbindung der Formel

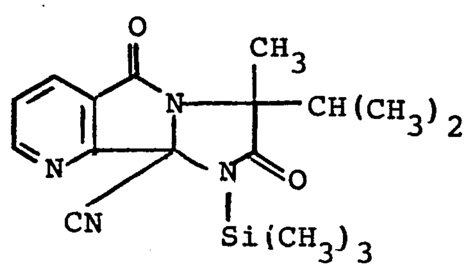

(1)

gemäß Anspruch 1.

5) Verbindung der Formel

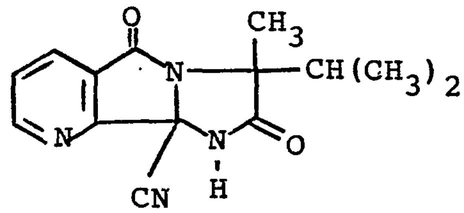

(2)

gemäß Anspruch 1.

6) Verbindung der Formel

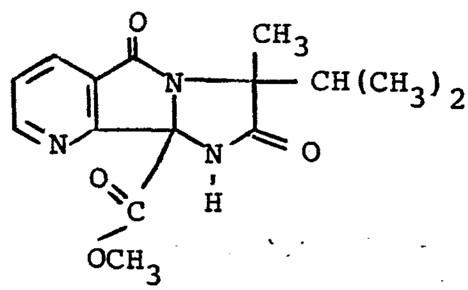

(3)

7) Verfahren zur Herstellung von Imidazo-pyrrolo-pyridin-Derivaten der Formel (I),

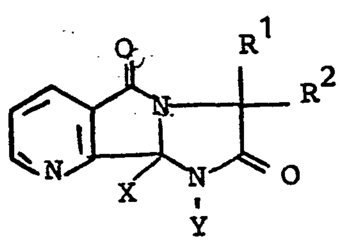

(I)

in welcher

$R^1$ und $R^2$ unabhängig voneinander für Alkyl stehen,

X für Cyano, Alkoxycarbonyl oder Aminocarbonyl steht und

Y für Wasserstoff steht oder für den Fall, daß X für Cyano steht, auch für die Trimethylsilylgruppe steht,

dadurch gekennzeichnet, daß man

(a) Imidazo- pyrrolo-pyridine der Formel (II)

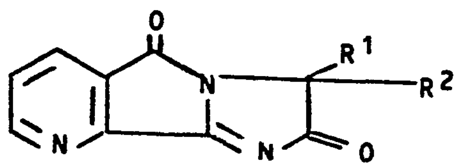

(II)

in welcher

$R^1$ und $R^2$ die oben angegebene Bedeutung haben,

mit Trimethylsilylcyanid der Formel (III),

$$(CH_3)_3Si - CN \qquad (III)$$

gegebenenfalls in Gegenwart eines Verdünnungsmittels umsetzt oder daß man

(b) die nach Verfahren (a) erhältlichen Imidazo-pyrrolo-trimethylsilyl-pyridine der Formel (Ia),

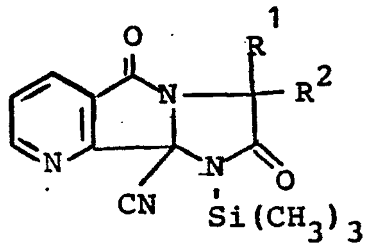

(Ia)

in welcher

$R^1$ und $R^2$ die oben angegebene Bedeutung haben,

durch Umsetzung mit Hydroxyverbindungen der Formel (IV),

$$R^3 - OH \qquad (IV)$$

in welcher

$R^3$ für Wasserstoff oder für Alkyl steht,

gegebenenfalls in Gegenwart eines Verdünnungsmittels und gegebenenfalls in Gegenwart eines Katalysators verseift.

8) Herbizide Mittel, gekennzeichnet durch einen Gehalt an mindestens einem Imidazo-pyrrolo-pyridin-Derivat der allgemeinen Formel (I) gemäß Anspruch 1.

9) Verwendung von Imidazo-pyrrolo-pyridin-Derivaten der allgemeinen Formel (I) gemäß Anspruch 1 zur Bekämpfung von unerwünschtem Pflanzenwachstum.

10) Verfahren zur Herstellung von herbiziden Mitteln, dadurch gekennzeichnet, daß man Imidazo-pyrrolo-pyridin-Derivate der allgemeinen Formel (I) gemäß Anspruch 1 mit Streckmitteln und/oder oberflächenaktiven Mitteln vermischt.

Europäisches Patentamt

# EUROPÄISCHER RECHERCHENBERICHT

0170860

Nummer der Anmeldung

EP 85 10 7933

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 041 623 (AMERICAN CYANAMID)<br>* Ansprüche 1,2 *<br>--- | 1,8 | C 07 D 471/14<br>C 07 F 7/10<br>A 01 N 43/90 //<br>(C 07 D 471/14<br>C 07 D 235:00<br>C 07 D 221:00<br>C 07 D 209:00 ) |
| A | GB-A-1 547 660 (AMERICAN CYANAMID)<br>* Anspruch 1; Beispiel 6 *<br>--- | 1,8 | |
| D,A | EP-A-0 133 309 (AMERICAN CYANAMID)<br>* Ansprüche 1,3 *<br>----- | 1,8 | |
| | | | RECHERCHIERTE SACHGEBIETE (Int. Cl.4) |
| | | | C 07 D 471/00<br>C 07 F 7/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 14-10-1985 | ALFARO I. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82